# EUROPÄISCHE PATENTSCHRIFT

(11) **EP 2 008 607 B1**
(45) Veröffentlichungstag und Bekanntmachung des Hinweises auf die Patenterteilung: **02.11.2011**
(21) Anmeldenummer: 08009665.4
(22) Anmeldetag: 28.05.2008
(51) Int. Cl.: A61B 19/00, A61B 17/00

(54) **Kupplungsvorrichtung zum Festlegen medizinischer Instrumente an einer Haltevorrichtung**
Coupling device for fixing medical instruments to a holding device
Dispositif de couplage destiné à fixer des instruments médicaux sur un dispositif de retenue

(30) Priorität: 29.06.2007 DE 102007030310
(43) Veröffentlichungstag der Anmeldung: 31.12.2008
(73) Patentinhaber: Karl Storz GmbH & Co. KG, 78532 Tuttlingen (DE)
(72) Erfinder: Hermle, Rainer, 78559 Gosheim (DE); Efinger, Andreas, 78604 Rietheim (DE)
(74) Vertreter: Hofmeister, Frank

(56) Entgegenhaltungen:
- EP-A- 0 418 620
- DE-A1- 10 357 104
- US-A- 5 674 225
- US-A1- 2007 270 640

## Beschreibung

Die Erfindung betrifft eine Kupplungsvorrichtung zum Festlegen medizinischer Instrumente an einer Haltevorrichtung mit einer an einem der miteinander zu verbindenden Bauteile ausgebildeten Kupplungsaufnahme und einem am anderen Bauteil ausgebildeten, in die Kupplungsaufnahme einsetzbaren Kupplungsstecker, wobei die Kupplungsaufnahme und der Kupplungsstecker in der miteinander gekoppelten Position über mindestens ein Sperrelement gegeneinander fixierbar sind.

Derartige Kupplungsvorrichtungen werden im medizinischen Bereich, beispielsweise bei endoskopischen Operationen, verwendet, um medizinische Instrumente, Geräte und/oder Zubehör an Haltevorrichtungen festzulegen, um die operierenden Ärzte und/oder deren Assistenten zu entlasten. Die Haltevorrichtungen erlauben dabei ein positionsgenaues Ausrichten der festgelegten Instrumente. Da es während der Operation notwendig sein kann, die befestigten medizinischen Instrumente oder dergleichen auszuwechseln, ist es erforderlich, dass die Kupplungssysteme einfach und schnell zu betätigen sind.

Eine gattungsgemäße Kupplungsvorrichtung ist beispielsweise aus der DE 203 17 693 U1 bekannt. Bei diesem bekannten Kupplungssystem erfolgt die Kopplung zwischen der Kupplungsaufnahme und dem Kupplungsstecker über ein in der Kupplungsaufnahme angeordnetes federbelastetes Rastelement, das in der gekoppelten Position rastend in eine am Kupplungsstecker ausgebildete Ringnut eingreift. Diese bekannte Konstruktion ermöglicht zwar auch eine schnelle und unkomplizierte Kopplung der miteinander zu verbindenden Bauteile, jedoch gewährleistet diese keine verdrehsichere Lagerung der beiden Bauteile zueinander. Darüber hinaus weisen federbelastete Rastelemente immer ein gewisses Kupplungsspiel auf und sind nur schlecht zu reinigen.

Davon ausgehend liegt der Erfindung die **Aufgabe** zugrunde, eine Kupplungsvorrichtung der eingangs genannten Art zu schaffen, die bei einfachem Aufbau einerseits eine lagesichere Festlegung des medizinischen Instruments ermöglicht und andererseits eine einfache und schnelle Handhabung gewährleistet.

Die **Lösung** dieser Aufgabenstellung ist erfindungsgemäß dadurch gekennzeichnet, dass die Kupplungsaufnahme als hülsenfömige, mehrere voneinander beabstandete Federarme aufweisende Spannzange ausgebildet ist und der Kupplungsstecker einen mit mindestens einem radial nach außen weisenden Rastnocken versehenen Kupplungszapfen aufweist, wobei der mindestens eine Rastnocken des Kupplungszapfens in der gekoppelten Position eine korrespondierende Hinterschneidung an der Spannzange rastend hintergreift, und dass das Sperrelement so auf die Spannzange aufschraubbar ist, dass die Federarme der Spannzange in der gekoppelten Position in Radialrichtung nach außen fixiert sind.

Durch die erfindungsgemäße Ausbildung von Kupplungsaufnahme und Kupplungsstecker als Spannzange und in der Spannzange verrastbarer Kupplungszapfen wird eine besonders einfach und schnell zu handhabende Kupplungsverbindung für die miteinander zu verbindenden Bauteile, nämlich das medizinische Instrument einerseits und die Haltevorrichtung andererseits, bereitgestellt. Die Sicherung dieser Rastkupplungsverbindung erfolgt ebenso einfach und schnell über das auf die Spannzange aufschraubbare Sperrelement, das in der miteinander gekoppelten Position der Bauteile ein radiales Öffnen der Spannzange verhindert.

Gemäß der Erfindung weist der Kupplungszapfen Vorsprünge auf, die in der gekoppelten Position mit korrespondierenden Vorsprüngen der Spannzange kämmen. Diese Vorsprünge dienen dazu, zusätzlich zu der Fixierung des Kupplungszapfens in Axialrichtung in der Spannzange aufgrund der Verrastung, eine gegenseitige Verdrehsicherung der Bauteile zu gewährleisten.

Um die Kupplung des Kupplungszapfens und der Spannzange zu erleichtern und sicherzustellen, dass der Kupplungszapfen und die Spannzange in der vorgesehenen Position ineinander greifen, sind erfindungsgemäß die in der gekoppelten Position aneinander anliegenden Flanken der Vorsprünge des Kupplungszapfens und der Spannzange abgeschrägt ausgebildet, so dass aufgrund der Abschrägungen eine automatische Selbstzentrierung der Bauteile zueinander stattfindet.

Weiterhin wird mit der Erfindung vorgeschlagen, dass der Kupplungszapfen des Kupplungssteckers und die als Spannzange ausgebildete Kupplungsaufnahme in der gekoppelten Position formschlüssig miteinander verbunden sind, um eine spielfreie und positionsgenaue Fixierung der Bauteile zueinander zu gewährleisten.

Gemäß einer ersten Ausführungsform der Erfindung wird vorgeschlagen, dass das Sperrelement als verdrehbar auf der Kupplungsaufnahme gelagerte Überwurfmutter ausgebildet ist.

Gemäß einer bevorzugten zweiten Ausführungsform der Erfindung wird vorgeschlagen, dass das Sperrelement aus einer auf die Spannzange aufschraubbaren Mutter und einem mit der Mutter in Wirkverbindung stehenden und in Axialrichtung auf die Spannzange aufsetzbaren Klemmring besteht.

Die das Öffnen der Spannzange verhindernde Klemmwirkung des Klemmrings kann erfindungsgemäß dadurch verstärkt werden, dass die der Spannzange zugewandte Innenseite des Klemmrings und die äußere Mantelfläche der Spannzange miteinander korrespondierend konisch ausgebildet sind. Diese Ausbildung der einander zugewandten Flächen als Konus und Gegenkonus bewirkt eine radial nach innen gerichtete formschlüssige Flächenpressung auf die Spannzange und verhindert so ein Öffnen der Spannzange und somit Freigeben des Kupplungszapfens.

Schließlich wird mit der Erfindung vorgeschlagen, dass die Spannzange und der Klemmring über mindestens ein Sicherungselement gegen ein Verdrehen relativ zueinander gesichert sind. Dieses mindestens eine Sicherungselement gewährleistet, dass der Klemmring beim Aufschrauben der Mutter auf die Spannzange sich nicht mit der Mutter um die Längsachse der Spannzange mitdreht, sondern die Drehbewegung der Mutter in eine Linearbewegung des Klemmrings in Axialrichtung der Spannzange umgewandelt wird.

Weitere Merkmale und Vorteile der Erfindung ergeben sich anhand der zugehörigen Zeichnung, in der ein Ausführungsbeispiel einer erfindungsgemäßen Kupplungsvorrichtung zum Festlegen medizinischer Instrumente an einer Haltevorrichtung nur beispielhaft dargestellt ist, ohne die Erfindung auf dieses Ausführungsbeispiel zu beschränken. In der Zeichnung zeigt:
- Fig. 1: eine perspektivische Explosionszeichnung einer erfindungsgemäßen Kupplungsvorrichtung zum Festlegen medizinischer Instrumente an einer Haltevorrichtung;
- Fig. 2: eine Seitenansicht der Kupplungsvorrichtung vor dem Koppeln von Kupplungsaufnahme und Kupplungsstecker;
- Fig. 3: eine um 90° gedrehte Seitenansicht der Kupplungsvorrichtung gemäß Fig. 2, jedoch Kupplungsaufnahme und Kupplungsstecker im gekoppelten Zustand darstellend und
- Fig. 4: einen Längsschnitt entlang der Linie IV-IV gemäß Fig. 3.

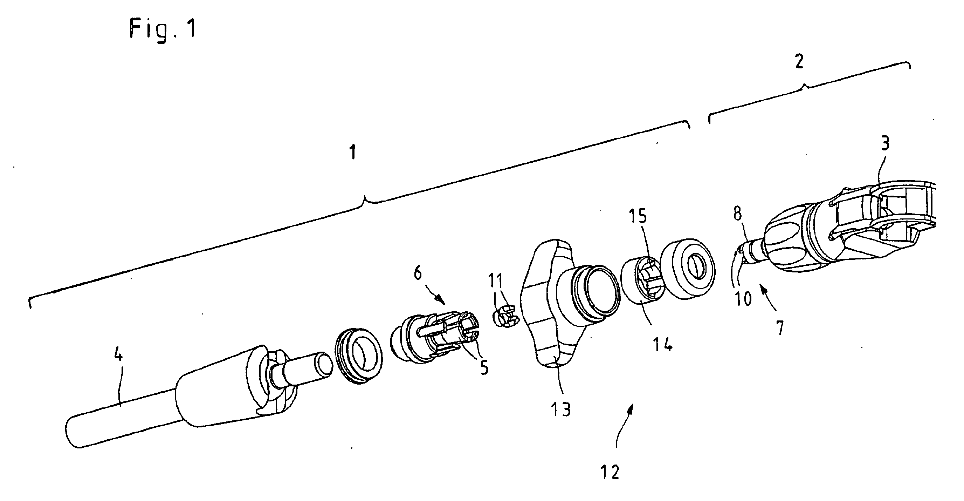

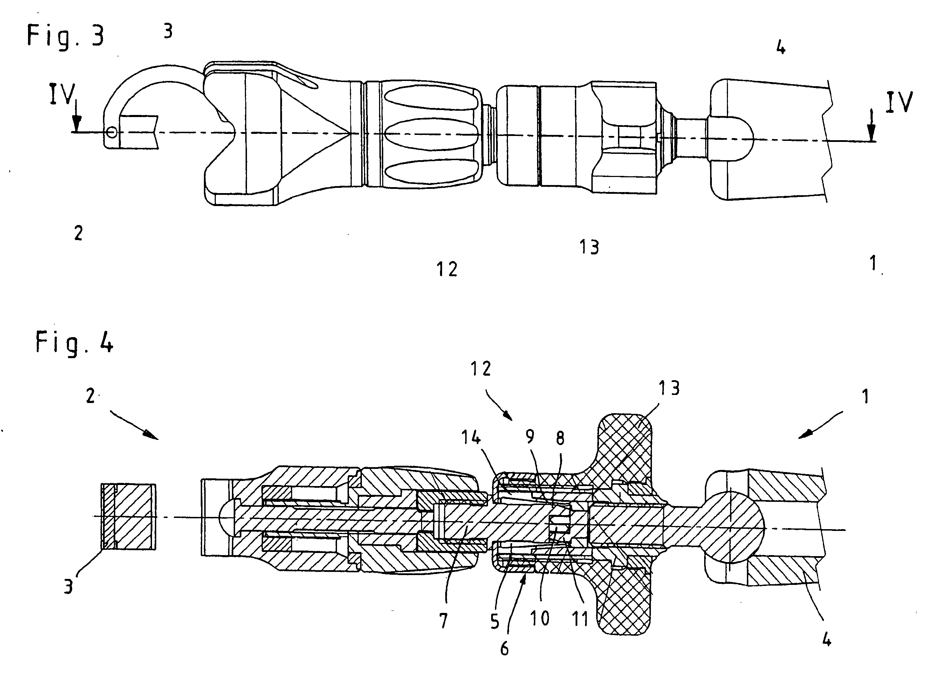

Die in den Abbildungen Fig. 1 bis 4 dargestellte Kupplungsvorrichtung zum Festlegen medizinischer Instrumente an einer Haltevorrichtung besteht im Wesentlichen aus einer Kupplungsaufnahme 1 und einem in die Kupplungsaufnahme 1 einsetzbaren Kupplungsstecker 2 zum Verbinden der beiden miteinander zu koppelnden Bauteile, nämlich einem medizinischen Instrument 3 einerseits und einer Haltevorrichtung 4 andererseits, wobei, wie in Fig. 1 bis 4 dargestellt, das Festlegen des medizinischen Instruments 3 an der Haltevorrichtung 4 auch indirekt über eine Instrumentenaufnahme 3 erfolgen kann, in der das medizinische Instrument 3 seinerseits festlegbar ist.

Derartige Kupplungsvorrichtungen werden verwendet, um bei Operationen zur Entlastung des Operationspersonals medizinische Instrumente positionsgenau an entsprechenden Haltevorrichtungen festlegen zu können.

Bei der dargestellten Ausführungsform ist der Kupplungsstecker 2 an der Instrumentenaufnahme 3 und die Kupplungsaufnahme 1 an der Haltevorrichtung 4 ausgebildet. Selbstverständlich ist es ohne die Funktion des Kupplungssystems zu beeinflussen auch möglich, die Kupplungsaufnahme 1 am medizinischen Instrument 3 bzw. der Instrumentenaufnahme 3 und den Kupplungsstecker 2 an der Haltevorrichtung 4 auszubilden.

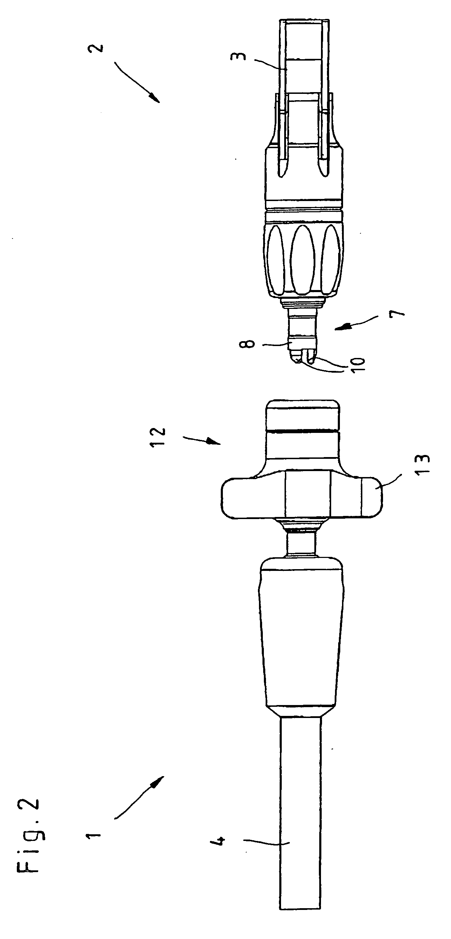

Wie insbesondere aus der Explosionszeichnung gemäß Fig. 1 ersichtlich, ist die Kupplungsaufnahme 1 als hülsenfömige, mehrere voneinander beabstandete Federarme 5 aufweisende Spannzange 6 ausgebildet. Die Federarme 5 umschließen den Aufnahmeraum, in dem der Kupplungsstecker 2 in der gekoppelten Position Aufnahme findet. Wie weiterhin aus Fig. 1 und 2 ersichtlich, ist der Kupplungsstecker 2 als in die Spannzange 6 einsetzbarer Kupplungszapfen 7 ausgebildet.

Um den Kupplungszapfen 7 des Kupplungssteckers 2 und die Spannzange 6 der Kupplungsaufnahme 1 nach dem Ineinanderstecken gegenseitig in Axialrichtung zu fixieren, weist der Kupplungszapfen 7 einen als Verdickung ausgebildeten radial nach außen weisenden Rastnocken 8 auf, der in der gekoppelten Position eine korrespondierende Hinterschneidung 9 an der Innenseite der Federarme 5 rastend hintergreift. Die Federelastizität der Federarme 5 sorgt dafür, dass die Federarme 5 der Spannzange 6 beim Einschieben des Kupplungszapfens 7 in die Spannzange 6 durch den Rastnocken 8 radial nach außen gedrückt werden und anschließend verrastend zurückfedern, sobald der Rastnocken 8 die Hinterschneidung 9 der Federarme 5 hintergreift.

Diese Verrastung des Rastnockens 8 hinter der Hinterschneidung 9 der Federarme 5 verhindert, dass die zusammengefügten Bauteile Kupplungszapfen 7 und Spannzange 6 in axialer Richtung wieder auseinander rutschen können.

Um zusätzlich ein Verdrehen der Bauteile Kupplungszapfen 7 und Spannzange 6 relativ zueinander zu verhindern und somit das medizinische Instrument 3 bzw. die Instrumentenaufnahme 3 positionsgenau in der Haltevorrichtung 4 lagern zu können, weist der Kupplungszapfen 7 in axialer Richtung vorstehende Vorsprünge 10 auf, die in der gekoppelten Position mit korrespondierenden Vorsprüngen 11 der Spannzange 6 kämmen. Durch dieses gegenseitige kämmende Eingreifen der Vorsprünge 10 des Kupplungszapfens 7 sowie der Vorsprünge 11 der Spannzange 6 blockieren sich die beiden Bauteile Kupplungszapfen 7 und Spannzange 6 gegenseitig und verhindern so ein gegenseitiges Verdrehen relativ zueinander um die Längsachse der Kupplungsvorrichtung.

Das Zusammenfügen des Kupplungszapfens 7 und der Spannzange 6 wird bei der dargestellten Ausführungsform dadurch erleichtert, dass die in der gekoppelten Position aneinander anliegenden Flanken der Vorsprünge 10 und 11 des Kupplungszapfens 7 und der Spannzange 6 abgeschrägt ausgebildet sind, so dass aufgrund der Abschrägungen eine automatische Selbstzentrierung der Bauteile zueinander stattfindet.

Wie weiterhin aus Fig. 1 und 2 ersichtlich, weist die Kupplungsvorrichtung darüber hinaus ein Sperrelement 12 auf, über das die Spannzange 6 und der Kupplungszapfen 7 in der gekoppelten Position gegeneinander derart fixierbar sind, dass das Sperrelement 12 ein Öffnen der Spannzange 6 durch Auseinanderbiegen der Federarme 5 verhindert.

Bei der dargestellten Ausführungsform der Kupplungsvorrichtung besteht das Sperrelement 12 aus einer auf die Spannzange 6 aufschraubbaren Mutter 13 und einem mit der Mutter 13 in Wirkverbindung stehenden und in Axialrichtung auf die Spannzange 6 aufschiebbaren Klemmring 14. Um zu verhindern, dass sich der Klemmring 14 beim Aufschrauben der Mutter 13 mit der Mutter 13 mitdreht, sind die Spannzange 6 und der Klemmring 14 über mindestens ein Sicherungselement 15, beispielsweise Vorsprünge und korrespondierende Aufnahmen, gegen ein Verdrehen relativ zueinander gesichert. Bei der dargestellten Ausführungsform ist die Mutter 13 als Drehgriff ausgestaltet, wodurch das Betätigen des Sperrelements 12 deutlich erleichtert wird.

Das Verbinden der Kupplungsaufnahme 1 mit dem Kupplungsstecker 2 einer Kupplungsvorrichtung gemäß Fig. 1 und 2 geschieht wie folgt:
Zum Festlegen des medizinischen Instruments 3 bzw. der Instrumentenaufnahme 3 an der Haltevorrichtung 4 wird der Kupplungszapfen 7 des bei dieser Ausführungsform an der Instrumentenaufnahme 3 ausgebildeten Kupplungssteckers 2 in die als Spannzange 6 ausgebildete Kupplungsaufnahme 1 der Haltevorrichtung 4 eingeschoben.

Durch den am vorderen Bereich des Kupplungszapfens 7 angeformten Rastnocken 8 werden die federelastischen Federarme 5 der Spannzange 6 beim Einschieben des Kupplungszapfens 7 in die Spannzange 6 radial nach außen gebogen, bis der Rastnocken 8 die auf der Innenseite der Spannzange 6 ausgebildete Hinterschneidung 9 hintergreift. Sobald der Kupplungszapfen 7 so weit in die Spannzange 6 eingeschoben wurde, federn die Rastarme 5 zurück in ihre ursprüngliche Position und fixieren so den Kupplungszapfen 7 in axialer Richtung gegen Herausfallen aus der Spannzange 6.

Zusätzlich zum Verrasten der Rastnocken 8 mit den Hinterschneidungen 9 laufen die Abschrägungen der am Kupplungszapfen 7 angeformten Svorsprünge 10 gegen die korrespondierenden Vorsprünge 11 der Spannzange 6 an und positionieren selbstzentrierend den Kupplungszapfen 7 und die Spannzange 6 verdrehsicher relativ zueinander.

Um zu verhindern, dass die Federarme 5 der Spannzange 6 radial nach außen gebogen werden können, beispielsweise aufgrund einer starken axialen Zugkraft entgegen der Einsteckrichtung des Kupplungszapfens 7, und so die Rastverbindung zwischen dem Kupplungszapfen 7 und der Spannzange 6 aufheben, wird nach dem Einschieben des Kupplungszapfens 7 und Verrasten mit der Spannzange 6 das Sperrelement 12 betätigt, um die Federarme 5 der Spannzange 6 in der gekoppelten Position gegen eine radial nach außen gerichtete Bewegung zu sichern.

Hierzu wird die mit dem Klemmring 14 in Wirkverbindung stehende Mutter 13 auf die Spannzange 6 aufgeschraubt, wobei die Rotation der Mutter 13 eine Verlagerung des Klemmrings 14 in Axialrichtung auf die Spannzange 6 bewirkt.

Die das Öffnen der Spannzange 6 verhindernde Klemmwirkung des Klemmrings 14 wird bei der dargestellten Ausführungsform dadurch verstärkt, dass die der Spannzange 6 zugewandte Innenseite des Klemmrings 14 und die äußere Mantelfläche der Spannzange 6 miteinander korrespondierend konisch ausgebildet sind. Diese Ausbildung der einander zugewandten Flächen als Konus und Gegenkonus bewirkt eine radial nach innen gerichtete formschlüssige Flächenpressung auf die Spannzange 6 und verhindert so ein Öffnen der Spannzange 6 und somit Freigeben des Kupplungszapfens 7.

Das Lösen der Kupplung erfolgt in umgekehrter Reihenfolge beginnend mit dem Lösen des Sperrelements 12. Beim Abschrauben der Mutter 13 von der Spannzange 6 wird der Klemmring 14 über die Mutter 13 von der Spannzange 6 herunter geschoben und gibt so die Federarme 5 der Spannzange 6 wieder frei. Sobald nun über den Kupplungszapfen 7 eine in axialer Richtung wirkende Zugkraft ausgeübt wird, drückt der Rastnocken 8 des Kupplungszapfens 7 die Federarme 5 der Spannzange 6 wieder radial nach außen, bis der Rastnocken 8 außer Eingriff mit der Hinterschneidung 9 der Spannzange 6 tritt und der Kupplungszapfen 7 nachfolgend wieder aus der Spannzange herausgezogen werden kann.

Eine solchermaßen ausgebildete Kupplungsvorrichtung zeichnet sich dadurch aus, dass sie bei einfachem Aufbau eine lagesichere Festlegung des medizinischen Instruments 3 bzw. der Instrumentenaufnahme 3 ermöglicht und darüber hinaus eine einfache und schnelle Handhabung gewährleistet.

### Bezugszeichenliste

- 1: Kupplungsaufnahme
- 2: Kupplungsstecker
- 3: medizinisches Instrument / Instrumentenaufnahme
- 4: Haltevorrichtung
- 5: Federarm
- 6: Spannzange
- 7: Kupplungszapfen
- 8: Rastnocken
- 9: Hinterschneidung
- 10: Vorsprung
- 11: Vorsprung
- 12: Sperrelement
- 13: Mutter
- 14: Klemmring
- 15: Sicherungselement

## Patentansprüche

1. Kupplungsvorrichtung zum Festlegen medizinischer Instrumente an einer Haltevorrichtung mit einer an einem der miteinander zu verbindenden Bauteile (3 oder 4) ausgebildeten Kupplungsaufnahme (1) und einem am anderen Bauteil (4 oder 3) ausgebildeten, in die Kupplungsaufnahme (1) einsetzbaren Kupplungsstecker (2), wobei die Kupplungsaufnahme (1) und der Kupplungsstecker (2) in der miteinander gekoppelten Position über mindestens ein Sperrelement (12) gegeneinander fixierbar sind, wobei die Kupplungsaufnahme (1) als hülsenfömige, mehrere voneinander beabstandete Federarme (5) aufweisende Spannzange (6) ausgebildet ist und der Kupplungsstecker (2) einen mit mindestens einem radial nach außen weisenden Rastnocken (8) versehenen Kupplungszapfen (7) aufweist, wobei der mindestens eine Rastnocken (8) des Kupplungszapfens (7) in der gekoppelten Position eine korrespondierende Hinterschneidung (9) an der Spannzange (6) rastend hintergreift, und dass das Sperrelement (12) so auf die Spannzange (6) aufschraubbar ist, dass die Federarme (5) der Spannzange (6) in der gekoppelten Position in Radialrichtung nach außen fixiert sind,
**dadurch gekennzeichnet,**
**dass** der Kupplungszapfen (7) Vorsprünge (10) aufweist, die in der gekoppelten Position mit korrespondierenden Vorsprüngen (11) der Spannzange (6) kämmen.

2. Kupplungsvorrichtung nach Anspruch 1, **dadurch gekennzeichnet, dass** die in der gekoppelten Position aneinander anliegenden Flanken der Vorsprünge (10, 11) des Kupplungszapfens (7) und der Spannzange (6) abgeschrägt ausgebildet sind.

3. Kupplungsvorrichtung nach Anspruch 1 oder 2, **dadurch gekennzeichnet, dass** der Kupplungszapfen (7) des Kupplungssteckers (2) und die als Spannzange (6) ausgebildete Kupplungsaufnahme (1) in der gekoppelten Position formschlüssig miteinander verbunden sind.

4. Kupplungsvorrichtung nach einem der Ansprüche 1 bis 3, **dadurch gekennzeichnet, dass** das Sperrelement (12) als verdrehbar auf der Kupplungsaufnahme (1) gelagerte Überwurfmutter ausgebildet ist.

5. Kupplungsvorrichtung nach einem der Ansprüche 1 bis 3, **dadurch gekennzeichnet, dass** das Sperrelement (12) aus einer auf die Spannzange (6) aufschraubbaren Mutter (13) und einem mit der Mutter (13) in Wirkverbindung stehenden und in Axialrichtung auf die Spannzange (6) aufsetzbaren Klemmring (14) besteht.

6. Kupplungsvorrichtung nach Anspruch 5, **dadurch gekennzeichnet, dass** die der Spannzange (6) zugewandte Innenseite des Klemmrings (14) und die äußere Mantelfläche der Spannzange (6) miteinander korrespondierend konisch ausgebildet sind.

7. Kupplungsvorrichtung nach Anspruch 5 oder 6, **dadurch gekennzeichnet, dass** die Spannzange (6) und der Klemmring (14) über mindestens ein Sicherungselement (15) gegen ein Verdrehen relativ zueinander gesichert sind.

## Claims

1. Coupling device for localizing medical instruments on a holding apparatus comprising a coupling receptacle (1) that is configured on one of the components (3 or 4) to be connected with each other and a coupling connector (2) that is configured on the other component (4 or 3) and that is insertable in the coupling receptacle (1), and wherein the coupling receptacle (1) and the coupling connector (2) can be fixed in position relative to each other in the connected position via at least one locking element (12), and wherein the coupling receptacle (1) is configured as a sleeve-shaped collet chuck (6) having several spring arms (5) that are disposed at a distance relative to each other, and wherein the coupling connector (2) comprises at least one coupling peg (7) having at least one latch cam (8) that is directed radially and outwardly, and wherein the at least one latch cam (8) of the coupling peg (7) engages and latches in the coupled position in a corresponding undercut (9) on the collet chuck (6), and wherein the locking element (12) can be screwed to the collet chuck in such a way that the spring arms (5) of the collect chuck (6), when in the coupled position, are fixed in the fastened position in radial direction toward the outside
**characterized in that**
the coupling peg (7) comprises projections (10) that mesh in the coupled position with corresponding projections (11) of the collet chuck (6).

2. Coupling device according to Claim 1 **characterized in that** the flanks of the projections (10, 11) of the coupling peg (7) and of the collet chuck (6) that are in contact with each other in the coupled position are configured as tapered.

3. Coupling device according to Claim 1 or 2 **characterized in that** the coupling peg (7) of the coupling connector (2) and the coupling receptacle (1) that is configured as a collet chuck (6) form a positive connection when in the coupled position.

4. Coupling device according to one of the Claims 1 to 3 **characterized in that** the locking element (12) is configured as a swivel nut that is rotably supported on the coupling receptacle (1).

5. Coupling device according to one of the Claims 1 to 3 **characterized in that** the locking element (12) comprises a nut (13) that can be screwed onto the collet chuck (6) and a clamping ring (14) that is in an active connection with the nut (13) and can be placed onto the collet chuck (6) in axial direction.

6. Coupling device according to Claim 5 **characterized in that** the inner side of the clamping ring (14) that is directed toward the collet chuck (6) and the outer jacket area of the collet chuck (6) are configured, corresponding to each other, as tapered.

7. Coupling device according to Claim 5 or 6 **characterized in that** the collet chuck (6) and the clamping ring (14) are secured relative to each other against displacement by a safety element (15).

## Revendications

1. Dispositif d'accouplement pour la fixation d'instruments médicaux sur un dispositif de support présentant une réception d'accouplement (1) formée sur un des éléments (3 ou 4) à raccorder entre eux et une fiche d'accouplement (2) formée sur l'autre élément (4 ou 3) et insérable dans la réception d'accouplement (1), la réception d'accouplement (1) et la fiche d'accouplement (2) pouvant être fixées l'une contre l'autre en position d'accouplement réciproque au moyen d'au moins un élément de verrouillage (12), la réception d'accouplement (1) étant réalisée comme pince de serrage (6) ayant la forme d'un manchon, comportant plusieurs bras de ressort (5) espacés l'un de l'autre et la fiche d'accouplement (2) comportant un tenon d'accouplement (7) pourvu d'au moins un ergot de verrouillage (8) s'étendant radialement vers l'extérieur, le ou les ergots de verrouillage (8) du tenon d'accouplement (7) venant en engagement de verrouillage derrière une contre-dépouille (9) correspondante sur la pince de serrage (6) dans la position d'accouplement, et l'élément de verrouillage (12) pouvant être vissé sur la pince de serrage (6) de manière à fixer les bras de ressort (5) de la pince de serrage (6) en direction radiale vers l'extérieur dans la position d'accouplement,
**caractérisé**
**en ce que** le tenon d'accouplement (7) présente des saillies (10) qui s'engrènent avec des saillies correspondantes (11) de la pince de serrage (6) dans la position d'accouplement.

2. Dispositif d'accouplement selon la revendication 1, **caractérisé en ce que** les flancs des saillies (10, 11) du tenon d'accouplement (7) et de la pince de serrage (6) reposant les uns contre les autres dans la position d'accouplement sont réalisés avec un chanfrein.

3. Dispositif d'accouplement selon la revendication 1 ou la revendication 2, **caractérisé en ce que** le tenon d'accouplement (7) de la fiche d'accouplement (2) et la réception d'accouplement (1) réalisée comme pince de serrage (6) sont raccordés l'un à l'autre par correspondance de forme dans la position d'accouplement.

4. Dispositif d'accouplement selon l'une des revendications 1 à 3, **caractérisé en ce que** l'élément de verrouillage (12) est réalisé comme écrou-chapeau monté de manière rotative sur la réception d'accouplement (1).

5. Dispositif d'accouplement selon l'une des revendications 1 à 3, **caractérisé en ce que** l'élément de verrouillage (12) se compose d'un écrou (13) pouvant être vissé sur la pince de serrage (6) et d'une bague de serrage (14) coopérant avec l'écrou (13) et pouvant être enfilée sur la pince de serrage (6) en direction axiale.

6. Dispositif d'accouplement selon la revendication 5, **caractérisé en ce que** la face intérieure de la bague de serrage (14) opposée à la pince de serrage (6) et la surface d'enveloppe extérieure de la pince de serrage (6) sont réalisées avec une forme conique pour correspondre l'une à l'autre.

7. Dispositif d'accouplement selon la revendication 5 ou la revendication 6, **caractérisé en ce que** la rotation de la pince de serrage (6) et de la bague de serrage (14) l'une par rapport à l'autre est empêchée par au moins un élément de blocage (15).
